# EUROPÄISCHE PATENTSCHRIFT

(11) **EP 1 706 230 B1**
(45) Veröffentlichungstag und Bekanntmachung des Hinweises auf die Patenterteilung: **09.02.2011**
(21) Anmeldenummer: 04804407.7
(22) Anmeldetag: 30.12.2004
(51) Int. Cl.: B21D 26/02, B21D 13/00, B21D 53/04

(54) **FLUID-UMFORMUNG VON METALLBLECHEN**
FLUID FORMING OF METAL SHEETS
DEFORMATION PAR FLUIDE DE TOLES METALLIQUES

(30) Priorität: 09.01.2004 DE 102004001418
(43) Veröffentlichungstag der Anmeldung: 04.10.2006
(73) Patentinhaber: Emitec Gesellschaft für Emissionstechnologie mbH, 53797 Lohmar (DE)
(72) Erfinder: HODGSON, Jan, 53842 Troisdorf (DE)
(74) Vertreter: Rössler, Matthias
(86) Internationale Anmeldenummer: PCT/EP2004/014822
(87) Internationale Veröffentlichungsnummer: WO 2005/065859

(56) Entgegenhaltungen:
- DE-A1- 10 026 679
- GB-A- 1 499 829
- US-A- 5 916 317

## Beschreibung

Die Erfindung betrifft ein Verfahren zur Herstellung einer Abgasbehandlungskomponente, die mindestens ein strukturiertes Metallblech aufweist gemäß dem Oberbegriff des Anspruchs 1. Weiterhin betrifft die Erfindung eine Vorrichtung zur Herstellung eines strukturierten Metallblechs, gemäß dem Oberbegriff des Anspruchs 11.

Abgasbehandlungskomponenten lassen sich hinsichtlich ihres Aufbaus in im wesentlichen zwei unterschiedliche Varianten unterteilen: extrudierte und zusammengesetzte. Bei den Abgasbehandlungskomponenten, die mit Hilfe des Extrusions-Verfahrens hergestellt werden, wird eine keramische oder metallische Masse in einem formbaren Zustand entsprechend der gewünschten Gestalt der Abgasbehandlungskomponente ausgebildet und anschließend gehärtet. Bei den Abgasbehandlungskomponenten, die aus einer Vielzahl von Einzelteilen zusammengesetzt sind, werden die Einzelteile (Bleche, Matten, Sensoren, Anschlüsse, Gehäuse, etc.) in der Regel einzeln hergestellt und anschließend zusammengesetzt, bevor diese dann miteinander verbunden werden. Die Erfindung betrifft nun vordergründig die letztgenannte Variante einer Abgasbehandlungskomponente.

Unter dem allgemeinen Begriff der Abgasbehandlungskomponente sind alle Komponenten zu verstehen, die derzeit gerade in mobilen Abgasanlagen eingesetzt werden. Dies schließt insbesondere katalytische Konverter, Filter, Partikelfallen, Wärmetauscher, Mischer, Adsorber und Mischformen daraus mit ein.

Die hier relevanten Abgasbehandlungskomponenten weisen mindestens ein strukturiertes Metallblech auf. "Metallblech" ist hier als ein Oberbegriff für Metallbänder, Blechscheiben, etc. zu verstehen. Das Metallblech besteht bevorzugt aus einem hochtemperaturfesten und korrosionsbeständigen Material. Insbesondere weist es eine Eisenbasis auf, wobei zumindest eines der Elemente aus der Gruppe Chrom, Nickel oder Aluminium ergänzend vorliegt. Bei dem Metallblech kann es sich sowohl um ein sogenanntes Hartblech als auch um ein sogenanntes Weichblech (welches nach dem Härten noch einmal weichgeglüht wurde) handeln. Die Dicke des Bleches liegt bevorzugt in einem Bereich kleiner 150 µm, insbesondere kleiner 110 µm und bevorzugt in einem Bereich von 20 µm bis 80 µm.

Ein solches strukturiertes Metallblech erfüllt in der Abgasbehandlungskomponente üblicherweise die Funktion, zumindest teilweise Strömungswege bzw. Kanäle durch die Abgasbehandlungskomponente hindurch zu begrenzen. Das strukturierte Metallblech wird dazu mit sich selbst bzw. weiteren Metallblechen zur Anlage gebracht und gegebenenfalls verbunden. Die daraus resultierenden Strömungswege bzw. Kanäle haben signifikante Auswirkungen auf die Wirkungsweise der Abgasbehandlungskomponente. So wird beispielsweise die pro Volumen der Abgasbehandlungskomponente bereitgestellte Oberfläche, der bei der Durchströmung der Abgasbehandlungskomponente resultierende Staudruck, die Durchmischung von Teilströmungen im Inneren der Abgasbehandlungskomponente, etc. maßgeblich beeinflusst. Dabei stehen die unterschiedlichen Anforderungen oft auch gegensinnig gegenüber, so dass die Verbesserung einer Eigenschaft zur Verschlechterung einer anderen führt. Bei der Optimierung der Strukturform des Metallblechs wurden daher bereits wesentliche Forschungsaktivitäten durchgeführt. Dabei hat sich beispielsweise herausgestellt, dass die resultierenden Strömungswege bzw. Kanäle relativ schlank mit steilen Flanken ausgebildet sein sollten. Dies führt nun dazu, dass die Struktur der Metallbleche nicht mehr zwangsläufig in Näherung einer normalen Sinus-Form entsprechen, sondern eine hiervon abweichende Struktur aufweisen. Außerdem hat sich herausgestellt, dass die Vorsehung unterschiedlicher Strukturen, beispielsweise einer Makro- und einer MikroStruktur, besonders vorteilhaft im Hinblick auf das Strömungsverhalten hat.

Die vorstehend genannten Anforderungen haben zur Folge, dass besonderes Augenmerk auch auf die Fertigung derartiger Metallbleche zur Ausbildung einer Struktur gerichtet sein muss. Üblicherweise werden derartige Metallbleche in Form eines Bandes miteinander kämmenden Profilwalzen zugeführt, die eine Deformierung bzw. Umformung des Bleches vornehmen. Aufgrund der Tatsache, dass für unterschiedliche Anwendungsfälle des Metallblechs jeweils verschiedene Parameter hinsichtlich der Struktur erzeugt werden müssen, sind bei der Herstellung mit Profilwalzen sehr viele, aufwendig hergestellte, teure Werkzeuge erforderlich. Weiter ist zu berücksichtigen, dass beim Hindurchführen der relativ dünnen Metallbleche durch die ineinandergreifenden Profilwalzen hindurch eine Beschädigung des Werkstoffs auftreten kann, da das Metallblech zum Teil über einen Zahn der Profilwalze gezogen wird. Eine solche Dickenreduzierung bzw. Rissbildung in dem Metallblech kann zu einem frühzeitigen Ausfall der Abgasbehandlungskomponente führen, weil diese Fehlstellen schließlich einen Angriffspunkt für die hohen thermischen und dynamischen Kräfte darstellen, die im z.B. Abgassystem eines Automobils auftreten.

Die gattungsgemäße GB-A-1,499,829 beschreibt ein Verfahren zu Herstellung von Auskragungen an Metallblechen. Dabei wird das Metallblech gegen ein weibliches Formteil zur Anlage gebracht und mittels eines Fluidstrahls an das Formteil gedrückt, bis das Metallblech an den Auskragungen reißt bzw. abschert.

Ferner ist aus der DE 100 26 679 A1 ein Verfahren zum Tiefziehen von Blechen und eine Vorrichtung zur Durchführung des Verfahrens bekannt. Hierbei wird zum Tiefziehen von ebenen Blechen ein randseitig eingespannte Blech mittels eines die Kontur des Formteils aufweisenden Formstempels gegen ein fluides Wirkmedium umgeformt.

Darüber hinaus gibt die US 5,916,317 ein Verfahren und eine Vorrichtung zur Herstellung von Metallbehältern an. Dabei wird ein Fluidstrom gegen die Innenwände der Behälter gerichtet.

Hiervon ausgehend ist es Aufgabe der vorliegenden Erfindung, eine Lösung für die oben genannten technischen Probleme anzugeben. Insbesondere ist es Aufgabe, ein Verfahren zur Herstellung strukturierter Metallbleche anzugeben, welches besonders einfach durchführbar, vielseitig einsetzbar, variabel und preiswert ist. Dabei sollen gleichzeitig die Anforderungen an eine Serienproduktion von strukturierten Metallblechen erfüllt sein. Zudem soll eine Vorrichtung zur Herstellung solcher strukturierten Metallbleche angegeben werden, die einfach aufgebaut und kostengünstig im Hinblick auf deren Herstellung und/oder Betrieb ist.

Diese Aufgaben werden gelöst durch ein Verfahren zur Herstellung einer Abgasbehandlungskomponente mit den Merkmalen des Patentanspruchs 1 bzw. einer Vorrichtung zur Herstellung einer Struktur in ein Metallblech mit den Merkmalen des Patentanspruchs 11 Weitere vorteilhafte Ausgestaltungen sind Gegenstand der jeweils abhängigen Patentansprüche.

Bei dem Verfahren zur Herstellung einer Abgasbehandlungskomponente, welche mindestens ein strukturiertes Metallblech umfasst, wird mindestens ein Metallblech mittels wenigstens einem Fluidstrom umgeformt, um das mindestens eine strukturierte Metallblech zu bilden, wobei das mindestens eine strukturierte Metallblech dann so angeordnet wird, dass ein Wabenkörper gebildet wird, der eine Vielzahl von Kanälen aufweist.

Unter "Umformen" wird insbesondere eine reine Biegebeanspruchung des Metallblechs verstanden. Ursache dafür ist der auf die Oberfläche des Metallblechs auftreffende Fluidstrom, welcher das Blech solange umformt, bis der Auftreffwinkel auf die Oberfläche des Metallblechs zu flach ist bzw. der umzuformende Abschnitt des Metallblechs vollständig in eine Gegenform gedrückt ist. Der Fluidstrom wird dabei in Abhängigkeit der herzustellenden Struktur, dem eingesetztten Fluid, dem Werkzeug und/oder dem Material des Metallblechs ausgebildet. Bevorzugt erfolgt auch eine Regelung des Fluidstromes in Abhängigkeit zumindest eines der vorstehend genannten Faktoren über die Anpassung zumindest eines der folgenden Parametern: Druck, Massenstrom, Strömungsgeschwindigkeit, Form, Formkraft. Bevorzugt werden hier zumindest einer der Parameter wie folgt gewählt:

| | |
|---|---|
| Druck: | größer 50 bar; insbesondere 60 bis 100 bar; |
| Volumenstrom: | größer 10 l/min pro 10 mm Düsenspaltbreite, insbesondere zwischen 15 und 30 l/min; |
| Strömungs-geschwindigkeit: | größer 100 m/s, insbesondere größer 150 m/s |
| Form: | kegelförmig; linien- bzw. spaltförmig, insbesondere konvergierend; |
| Umformkraft: | ca. 350 N bis 500 N. |

Die obenstehenden Werte betreffen insbesondere nicht-weichgeglühte Metallbleche (sogenanntes "Hartband") mit einer Blechdicke von ca. 50 µm und einer Breite von 75 mm, wobei das Hartband über die gesamte Breite gleichzeitig und gleichmäßig umgeformt wird. Aufgrund der Tatsache, dass die vorstehenden Parameter stark von der Fluidverteileinrichtung abhängig sind, die einen solchen Fluidstrom produziert, können die angegebenen Werte bei unterschiedlichen Vorrichtungen zur Durchführung des Verfahrens unter Umständen auch abweichen.

Bei dem erfindungsgemäßen Verfahren werden mehrere Metallbleche miteinander gestapelt, gewunden und/oder aufgerollt, wobei zumindest ein Teil davon strukturiert ist. Damit ist auch gemeint, dass ein Metallblech Bereiche mit einer Struktur und Bereiche ohne eine (solche) Struktur aufweisen kann, ebenso wie das separate glatte und strukturierte Metallbleche miteinander zu einem Wabenkörper angeordnet werden. Alternativ oder in Kombination dazu kann das mindestens eine strukturierte Metallblech mit weiteren Komponenten zusammengefügt werden, beispielsweise Fasermatten, Lochplatten, Isoliermatten, Dichtfolien etc..

Man unterscheidet vor allem zwei typische Bauformen für metallische Wabenkörper. Eine frühe Bauform, für die die DE 29 02 779 A1 typische Beispiele zeigt, ist die spiralige Bauform, bei der im wesentlichen eine glatte und eine gewellte Blechlage aufeinandergelegt und spiralförmig aufgewickelt werden. Bei einer anderen Bauform wird der Wabenkörper aus einer Vielzahl von abwechselnd angeordneten glatten und gewellten oder unterschiedlich gewellten Blechlagen aufgebaut, wobei die Blechlagen zunächst einen oder mehrere Stapel bilden, die miteinander verschlungen werden. Dabei kommen die Enden aller Blechlagen außen zu liegen und können mit einem Gehäuse oder Mantelrohr verbunden werden, wodurch zahlreiche Verbindungen entstehen, die die Haltbarkeit des Wabenkörpers erhöhen. Typische Beispiele dieser Bauformen sind in der EP 0 245 737 B1 oder der WO 90/03220 beschrieben. Auch seit langem bekannt ist es, die Blechlagen mit zusätzlichen Strukturen auszustatten, um die strömung zu beeinflussen und/oder eine Quervermischung zwischen den einzelnen Strömungskanälen zu erreichen, vgl. z.B. WO 91/01178. All diese Bauformen lassen sich mit dem hier vorgeschlagenen Verfahren sowie der später erläuterten Vorrichtung herstellen; deshalb Kann der Offenbarungsinhalt der vorgenannten Veröffentlichungen zur Erläuterung herangezogen werden.

Im Hinblick auf die Anzahl solcher Kanäle wird üblicherweise die sogenannte "Kanaldichte" herangezogen, die beschreibt, wie viele Kanäle in einer Einheitsquerschnittsfläche des Wabenkörpers vorliegen. Bevorzugt weist der gebildete Wabenkörper eine solche Kanaldichte von 100 cpsi ("cells per square inch"; Kanäle pro Quadratinch) bis 1600 cpsi auf. Die Kanäle verlaufen dabei im wesentlichen parallel zueinander und bilden separate, zumindest teilweise getrennte, von Kanalwänden umgebene, Strömungswege. Das Metallblech bzw. die weiteren Komponenten können zudem Öffnungen und/oder Leitstrukturen aufweisen, die eine Verbindung benachbart zueinander angeordneter Kanäle bereitstellen, so dass die in den Kanälen befindlichen Teilströmungen im Wabenkörper miteinander gemischt werden können. Üblicherweise verlaufen diese Kanäle im wesentlichen geradlinig von einer Stirnfläche des Wabenkörpers hin zu der gegenüberliegenden Stirnseite. Es sind jedoch auch Wabenkörper bekannt, deren Strömungswege einen hiervon abweichenden Verlauf aufweisen, beispielsweise wendelförmig, stufig, etc.

Gemäß einer weiteren Ausgestaltung des Verfahrens wird vorgeschlagen, dass der mindestens eine Fluidstrom zumindest einen der folgenden Bestandteile aufweist: Wasser, Öl, Partikel, Additive. Der Einsatz eines Fluidstromes, der im wesentlichen aus Wasser besteht, hat insbesondere im Hinblick auf Umweltaspekte bzw. kostengünstige Entsorgung bzw. Reinigung des Fluides Vorteile. Bei anderen Anwendungsgebieten, beispielsweise bei hohen Umformgraden, kann es auch vorteilhaft sein, einen Fluidstrom einzusetzen, der überwiegend aus Öl besteht. Dem Fluidstrom können zudem Partikel beigefügt sein, die z. B. zur Oberflächenbehandlung des Metallblechs beitragen (Induzierung von Eigenspannung, Reinigung, etc.). Zusätzlich können auch Additive beigemengt sein, die insbesondere chemische Substanzen umfassen, die z. B. zur Reinigung der Oberfläche des Metallblechs, der Reinigung der Fluidverteileinrichtung und/oder weiteren Komponenten der Vorrichtung zur Herstellung des strukturierten Metallblechs dienen. Weiter wird auch vorgeschlagen, dass das Verfahren intermittierend abläuft. Damit wird insbesondere auf den Vorschub des Metallblechs bei der Bearbeitung abgestellt. Zwar kann das Verfahren auch kontinuierlich durchgeführt werden, bevorzugt ist jedoch ein intermittierender Vorschub des Metallblechs. Die Fluidzufuhr bzw. die Ausbildung des zur Umformung erforderlichen Fluidstromes kann von dem intermittierenden Vorschub des Metallblechs abhängig gestaltet sein, so das sich mit der Frequenz des Vorschubs auch die Ausbildung des Fluidstromes überlagert. Es ist jedoch auch möglich, dass der Fluidstrom kontinuierlich bzw. permanent während des intermettierenden Vorschubes des Metallblechs aufrechterhalten wird.

Aufgrund der Tatsache, dass das hier beschriebene Verfahren vielfach im Rahmen einer Serienfertigung der Automobilzulieferbranche eingesetzt werden soll, wird ein Vorschub des Metallblechs von mindestens 10 m/min, insbesondere mindestens 12 m/min und bevorzugt mindestens 15 m/min eingestellt. Dadurch, dass sich die herzustellenden Strukturen in der Regel nach einem Muster wiederholen und die Herstellung einer Struktur bzw. eines Strukturabschnittes durch den Fluidstrom jeweils bei stillstehendem Metallblech vorgenommen wird, ergeben sich entsprechende Taktfrequenzen. Wird beispielsweise ein Blechabschnitt zur Generierung eines Strukturabsehnittes von einer Länge von beispielsweise 5 mm vom wenigstens einen Fluidstrom bearbeitet, so ergibt sich eine Taktfrequenz von ca. 40 Hz [¹/sekunde], wobei ein Vorschub des Metallblechs von ca. 12 m/min angenommen wurde. Für die Serienfertigung geeignete Verfahren weisen eine Taktfrequenz von mindestens 20 Hz auf. Vorteilhafterweise ist dieses Verfahren mit einer Vorschubgeschwindigkeit des Metallblechs von mindestens 20 m/min und insbesondere sogar von über 50 m/min zu betreiben.

Gemäß einer weiteren Ausgestaltung des Verfahrens wird das mindestens eine Metallblech vor der Umformung einem Verfahrensschritt umfassend eine Blechtrennung unterzogen. Die Blechtrennung kann beispielweise von einem der Fertigungsverfahren Schneiden, Stanzen oder auch durch hochenergetisches Strahlen mit einem Medium (Wasser, Abrasiv-Mittel, Laser, etc.) durchgeführt werden. In diesem Zusammenhang ist insbesondere keine vollständige Abtrennung von Metallblechen von einem Band bzw. Coil zu verstehen, sondern vielmehr die Durchführung einer Blechtrennung in bestimmten innenliegenden Bereichen des Metallblechs. Dadurch werden Öffnungen, Schlitze oder sonstige Aussparungen in das (noch glatte) Metallblech eingebracht. Die Positionierung derartiger Trennschnitte erfolgt ggf. im Hinblick auf die anschließend durchgeführte Blechumformung, so dass definierte Ausstülpungen in die das Metallblech eingebracht werden können. Weiter ist es auch möglich, diese Öffnungen zu Zwecken der Materialersparnis einzusetzen, wodurch zum Beispiel auch eine reduzierte Wärmekapazität erreicht wird. Ungeachtet dessen kann der Verfahrensschritt in einer Blechumtrennung dem Umformschritt auch noch einmal nachgeschaltet sein, beispielsweise um die Metallbleche auf die gewünschte Länge bzw. Breite zurechtzuschneiden, wie sie anschließend in der Abgasbehandlungskomponente vorliegen sollen.

Besonders bevorzugt wird ein im wesentlichen glattes Metallblech durch den wenigstens einen Fluidstrom in mindestens eine Gegenform gedrückt, so dass sich die Struktur des mindestens einen strukturierten Metallblechs ausbildet. Unter einer "Gegenform" werden insbesondere Gesenke, Matritzen, Profilwalzen, Zahnräder, etc. verstanden. Sie stellen eine Art Negativform dar, in die das Metallblech mit Hilfe des Fluidstromes hineingedrückt wird, sich daran anschmiegt und auf diese Weise die Struktur dauerhaft annimmt. Dabei findet in überwiegendem Maße eine plastische Umformung des Metallblechs statt. Die Gegenform weist dann eine Oberfläche auf, die im wesentlichen der gewünschten Struktur des Metallblechs entspricht. Erste Tests haben gezeigt, dass hier eine sehr geringe Formabweichung erreicht werden kann, das Metallblech die Kontur der Gegenform also sehr exakt annimmt. Die Gegenform kann dabei einteilig oder auch mehrteilig ausgeführt sein, wobei sich bei der letztgenannten Variante insbesondere auch Hinterschneidungen oder ähnliche Ausprägungen der Oberflächenstruktur gestalten lassen. Durch die Umformung bzw. das Eindrücken des Metallblechs mittels des Fluidstromes sind auch solch komplexe Gegenformen nachbildbar.

Entsprechend eine Weiterbildung des Verfahrens werden mindestens zwei Fluidströme eingesetzt, wobei ein erster Fluidstrom eine Blechumformung und ein nachgeschalteter zweiter Fluidstrom eine Fixierung des Metallblechs bezüglich der mindestens einen Gegenform bewirkt. Um sicherzustellen, dass das Metallblech bei der Blechumformung seine definierte Lage zu der Gegenform nicht verliert, wird hier vorgeschlagen, dass der bereits strukturiert ausgeformte Teilbereich des Metallblechs durch Einsatz eines zweiten Fluidstromes (Halte-Fluidstrom) im wesentlichen formschlüssig zu der Gegenform angeordnet bleibt. Damit kann ausgeschlossen werden, dass bereits bearbeitete Teilbereiche hin zum ersten Fluidstrom zurückgezogen werden.

Im Hinblick auf die mindestens eine Gegenform ist es besonders vorteilhaft, wenn diese gleichzeitig das mindestens eine strukturierte Metallblech in eine Vorschubrichtung transportiert. Das bedeutet, dass die Gegenform selbst den Antrieb bzw. das Transportorgan für den Transport des Metallblechs durch die Vorrichtung zur Herstellung einer Struktur in ein Metallblech hindurch darstellt. Dabei greift die hergestellte Struktur des Metallblechs mit der Oberfläche der Gegenform ineinander und eine Relativbewegung der Oberfläche der Gegenform zur Fluidverteileinrichtung führt somit auch zur Erzeugung einer Relativbewegung des mindestens einen strukturierten Metallblechs zur Fluidverteileinrichtung. Die Relativbewegung kann linearer und/oder rotatorischer Natur sein. Bei anderen Ausgestaltungen des Verfahrens kann der Transport des Metallblechs durch den mindestens einen Fluidstrom unterstützt oder sogar übernommen werden. Es ist auch möglich, dass der mindestens eine Fluidstrom den Vorschub bewirkt, die Gegenform aber die Vorschubgeschwindigkeit in einem vorgebbaren Bereich hält, indem sie den mit dem mindestens einen Fluidstrom bewirkten Vorschub bremst.

Gemäß einer Weiterbildung des Verfahrens, bei dem in einem der Blechumformung vorgelagerten Verfahrensschritt mittels Blechtrennung eine Mehrzahl von Schlitzen in dem mindestens einen Metallblech erzeugt wird, wird auch vorgeschlagen, dass eine kontrollierte Positionierung der Schlitze gegenüber der Gegenform durchgeführt wird. Das heißt mit anderen Worten, dass sichergestellt ist, dass die produzierten Schlitze im Metallblech auch an den gewünschten Orten der Gegenform positioniert sind, wenn die Umformung des Metallblechs stattfindet. Die Schlitze sind insbesondere zur teilweisen Begrenzung von Ausstülpungen, Leitflächen oder ähnlichen Vorsprüngen in das Metallblech eingebracht. Genau diese Ausstülpungen etc. werden durch besondere, hierfür geeignete Vorsprünge in der Gegenform gebildet, wobei sichergestellt ist, dass der Schlitz an der Stelle des jeweiligen Vorsprungs positioniert wird. Dies kann beispielsweise durch eine besondere Sensorik und/oder durch (mechanisch, elektrisch, EDV-technisch, etc.) gekoppelte Antriebe der Vorrichtung zur Durchführung der Blechtrennung und der Vorrichtung zur Durchführung der Blechumformung gewährleistet werden.

Nach einer weiteren Ausgestaltung des Verfahrens wird das mindestens eine strukturierte Metallblech nach dem Umformen einem Reinigungsprozess unterzogen. Dieser Reinigungsprozess, der gegebenenfalls auch mehrstufig aufgebaut sein kann, dient bevorzugt primär der Entfernung von Bestandteilen des Fluidstromes bzw. den auf die Oberfläche des Metallblechs befindlichen Verschmutzungen etc. Der Reinigungsprozess kann eine Wärmebehandlung, eine chemischen Behandlung und/oder auch eine mechanische Behandlung des Metallblechs umfassen. So kann bei einem Wasser-Fluidstrom die Oberfläche bzw. das Metallblech selbst durch Einsatz eines Ofens oder eines Gebläses getrocknet werden. Für Öl und/oder Verschmutzungen bieten sich entsprechende Maßnahmen an. Diese können das Bürsten, Ätzen oder sonstige Reinigungsprozesse umfassen.

In diesem Zusammenhang ist es besonders vorteilhaft, den so gebildeten Wabenkörper mit einem Gehäuse in Kontakt zu bringen und anschließend einen thermischen Fügeprozess durchzuführen. Grundsätzlich ist es möglich, mehrere solcher Wabenkörper in einem einzelnen Gehäuse anzuordnen, es ist aber auch möglich, dass ein Wabenkörper mit mehreren Gehäusen in Kontakt steht bzw. von mehreren Gehäusen in radialer Richtung zumindest teilweise umgeben ist. Nachdem der Wabenkörper in der gewünschten Position im Gehäuse angeordnet ist, wird hier ein thermischer Fügeprozess zur Anbindung der Komponenten des Wabenkörpers miteinander sowie des Wabenkörpers mit dem mindestens einen Gehäuse vorgeschlagen. Diese thermischen Fügeprozesse können zu Diffusions-, Schweiß- und/oder Lötverbindungen führen. Zusätzlich können dabei Beschichtungen auf dem mindestens einen Metallblech bzw. anderen Komponenten des Wabenkörpers generiert werden, was jedoch zusätzlich bzw. alternativ dazu in einem nachträglichen Beschichtungsprozess durchgeführt werden kann.

Gemäß einem weiteren Aspekt der Erfindung wird eine Vorrichtung zur Herstellung einer Struktur in ein Metallblech vorgeschlagen, wie mit den Merkmalen des Patentanspruchs 11 angegeben. Sie weist eine Fluidbereitstellungsvorrichtung, mindestens eine Fluidverteileinrichtung und mindestens eine Gegenform auf. Die Vorrichtung ist so ausgeführt, dass die mindestens eine Fluidverteileinrichtung zumindest einen ersten Fluidstrom und einen zweiten Fluidstrom bereitstellt. Die hier beschriebene Vorrichtung dient dazu , nach einem der vorgenannten erfindungsgemäßen Verfahren die Herstellung einer Struktur in einem Metallblech durchzuführen. Den zwei Fluidströmen kommen dabei vorzugsweise unterschiedliche Funktionen bei der Blechumformung zu. Während der erste Fluidstrom beispielsweise primär die Umformung des Metallblechs vornimmt, dient der zweite Fluidstrahl vorzugsweise vordergründig dem Niederhalten bzw. dem Anpressen des umgeformten Metallblechs an bzw. in der Gegenform. Dadurch wird stets eine definierte Lage des Metallblechs zu der Gegenform gewährleistet, so dass eine exakte Abbildung der Struktur der Gegenform im Metallblech gebildet ist. Der zweite Fluidstrahl kann darüber hinaus auch zum Nachkalibrieren bzw. für einen zweiten Umformschritt (beispielsweise bei Hinterschneidungen, etc.) eingesetzt werden. Vorteilhafterweise werden der erste Fluidstrom und der zweite Fluidstrom zumindest teilweise während der Bearbeitung gleichzeitig erzeugt, voneinander unterschiedliche Startzeitpunkte bzw. Endzeitpunkte sind jedoch unter Berücksichtigung der Ausprägung des Metallblechs gegebenenfalls wählbar. Grundsätzlich ist auch möglich, dass die Fluidverteileinrichtung gleichartige Medien mit dem Metallblech in Kontakt bringt, alternativ dazu können der erste Fluidstrom und der zweite Fluidstrom auch aus unterschiedlichen bzw. veschiedenartig ausgeprägten Medien gebildet sein. Die Fluidbereitstellungsvorrichtung dient dazu, die Fluidverteileinrichtung mit den entsprechenden Fluiden (Wasser, Öl, etc.) zu versorgen, so dass die Fluidströme mit den gewünschten Parametern generierbar sind. Teil dieser Fluidbereitstellungsvorrichtung können Rohrleitungen, Pumpen, Ventile, Messgeräte, etc. sein.

Erfindungsgemäß ist die Vorrichtung mit mindestens einem Sensor und wenigstens einer Auswerteeinheit ausgestattet. Derartige Sensoren können zur Funktionsüberwachung bzw. zur Lokalisierung von Arbeitsergebnissen eingesetzt werden. Die mit dem mindestens einem Sensor erzeugten Signale werden vorzugsweise in einer übergeordneten Auswerteeinheit zusammengefasst, die z.B. die Antriebe der unterschiedlichen Vorrichtungen aufeinander abstimmt.

In diesem Zusammenhang ist es besonders vorteilhaft, dass die mindestens eine Fluidverteileinrichtung Mittel zur Variierung wenigstens einem der folgenden Parameter hinsichtlich zumindest des ersten Fluidstromes oder des zweiten Fluidstromes hat:
a) Druck
b) Strömungsgeschwindigkeit
c) Volumenstrom
d) Form.

Grundsätzlich kann die Vorrichtung mit mehreren Fluidverteileinrichtungen ausgestattet sein, die beispielsweise zeitgleich nebeneinander über eine bestimmte Breite des Metallblechs einwirken, oder die in Vorschubrichtung des Metallblechs hintereinander angeordnet sind und gegebenenfalls zur Ausbildung unterschiedlicher Strukturen in das eine Metallblech dienen (z.B. Makrostruktur/Mikrostruktur). Zumindest eine dieser Fluidverteileinrichtungen, bevorzugt jede dieser Fluidverteileinrichtungen, weist solche Mittel zur Variierung der Parameter der Fluidströme auf. Die Variierung kann dabei unabhängig für jeden einzelnen Fluidstrom einer Fluidverteileinrichtung vorgenommen werden, aber auch eine gemeinsame Variierung des ersten Fluidstromes und des zweiten Fluidstromes ist möglich. Vorteilhaft ist es auch, wenigstens zwei der genannten Parameter a) bis d) varrieren zu können.

Bevorzugt werden diese Parameter während der Herstellung des strukturierten Metallblechs überwacht und gegebenenfalls nachgeregelt. Die genannten Mittel können Teil der Fluidverteileinrichtung selbst sein, es ist jedoch auch möglich, dass sie mittelbar mit der Fluidverteileinrichtung verbunden sind. Der Druck und der Volumenstrom können dabei beispielsweise gemeinsam mit der Fluidbereitstellungsvorrichtung überwacht bzw. korrigiert und/oder eingestellt werden. Die Strömungsgeschwindigkeit sowie die Form der Fluidströme lässt sich beispielsweise durch die Form der Austrittsöffnung der Fluidverteileinrichtung beeinflussen. Mit "Form" des Fluidstromes ist insbesondere die Austrittsart aus der Fluidverteileinrichtung gemeint, beispielsweise ob der Fluidstrom sich verjüngt, sich erweitert, ob er punktförmig, linienförmig oder flächig auf das Metallblech aufgegeben wird.

Gemäß einer Weiterbildung der Vorrichtung umfasst die mindestens eine Fluidverteileinrichtung wenigstens eine Düse mit einem Spalt. Grundsätzlich kann eine Düse sehr verschieden aufgebaut und an den jeweiligen Einsatzzweck bzw. die gewünschte Gestalt des strukturierten Metallblechs angepasst ausgeführt sein. Dabei kann unter einer "Düse" z. B. ein konisch zulaufendes Rohrstück zur Verengung von Rohrleitungen verstanden werden. Üblicherweise hat eine Düse die Eigenschaft, eine Beschleunigung des durch sie hindurchströmenden Fluids zu bewirken, was in der Regel mit einem Druckabfall einhergeht. Düsen können zum Zerstäuben von Fluiden, zum dispersen Vermengen verschiedener Fluide bzw. weiterer Komponenten und zu anderen Zwecken eingesetzt werden. Hier wird bevorzugt eine sich hin zu einem Spalt verjüngende Leitungskonstruktion vorgeschlagen. Dies geschieht mit besonderer Rücksicht auf die herzustellende Struktur, die wegen der üblicherweise gradlinigen Ausbildung von Kanälen ebenfalls linienartig aufgebaut ist. Der Spalt der Düse ist dabei bevorzugt im wesentlichen parallel zu den Linien der Maximal- bzw. Minimal der Struktur des Metallblechs bzw. der Gegenform ausgerichtet, um eine möglichst gleichmäßige Umformung eines Metallblech-Abschnitts zu ermöglichen. Werden hiervon abweichende Strukturen erzeugt bzw. zusätzliche Strukturen eingebracht, die lokal auf bestimmte Bereiche des Metallblechs begrenzt sind, können andere Düsenarten eingesetzt bzw. vorgesehen sein. Der Spalt hat bevorzugt eine Spaltbreite, die kleiner 1,0 mm beträgt, insbesondere kleiner 0,5 mm. Die Länge des Spaltes orientiert sich im wesentlichen an der Breite des Metallbleches, das zu bearbeiten ist. Üblicherweise wird vorgeschlagen, dass Metallbleche mit einer Breite bis zu 100 mm mit einer einzigen Spaltdüse bearbeitet werden, wobei diese dann eine der Breite des Metallblechs entsprechende Spaltlänge hat.

Besonders vorteilhaft ist es, wenn die mindestens eine Fluidverteileinrichtung zwei, von einem Mittelsteg gegenüberliegende Düsen mit jeweils einem Spalt aufweist. Eine solche Konstruktion ermöglicht, dass die Spalte der Düsen sehr dicht zueinander angeordnet sein können. Das erlaubt die schonende und gut reproduzierbare Herstellung besonders kleiner Strukturen im Metallblech, da die Fluidströme direkt auf benachbarte Strukturen der Gegenform bzw. des Metallblechs hin ausgerichtet werden können. Die beiden Spalte bzw. die beiden Düsen können dabei im wesentlichen symmetrisch zum Mittelsteg gestaltet sein, es ist jedoch auch möglich, dass diese insbesondere in der Nähe des Spaltes unter Berücksichtigung ihrer unterschiedlichen Funktion voneinander verschieden ausgeführt sind.

Gemäß einer weiteren Ausgestaltung der Vorrichtung wird vorgeschlagen, dass die mindestens eine Fluidverteileinrichtung Mittel zum Entfernen des Fluides nach dem Kontakt mit dem Metallblech umfasst. Versuche haben gezeigt, dass der auf das Metallblech aufgebrachte Fluidstrom auf der Oberfläche des glatten Teiles des Metallblechs haftet und an diesem entlangströmt. Dabei entfernt sich das Fluid sehr weit von der Bearbeitungsstelle, wodurch die Entsorgung bzw. die Wiederverwertung des Fluides deutlich erschwert wird. Deshalb wird hier vorgeschlagen, eine Umlenkung bzw. Entfernung des Fluidstromes nahe der Bearbeitungsstelle zu gewährleisten. Die dazu erforderlichen Mittel können beispielsweise Aufnahmekanäle in der Fluidverteileinrichtung, Leitbleche, Gebläse, Saugleitungen, etc. umfassen. Bevorzugt handelt es sich dabei um eine besondere Art der Fluidstrom-Führung durch Bauteile bzw. Teilbereiche der Fluidverteileinrichtung selbst. Auf diese Weise kann der Einsatz weiterer Wirkmedien (z. B. Druckluft, Vakuum) vermieden werden.

Weiter wird auch vorgeschlagen, dass die mindestens eine Gegenform ein drehbares Konturrad ist. Das Konturrad ist bevorzugt im wesentlichen rund und über einen Antrieb geregelt drehbar. Das Konturrad kann einteilig oder auch mehrteilig ausgebildet sein, beispielsweise als Scheibenanordnung. Das Konturrad hat über seine Breite die gewünschte Oberfläche, die letztendlich eine Negativform für die herzustellende Struktur im Metallblech darstellt. Als Konturen kommen Zacken, Zähne, Bolzen, Vorsprünge, etc. zum Einsatz, die vorzugsweise gleichmäßig über den Umfang des Konturrades hervorstehen.

In diesem Zusammenhang ist es besonders vorteilhaft, dass die Vorrichtung Mittel zum kontrollierten Antrieb des Konturrades in Abhängigkeit wenigstens eines der folgenden Faktoren hat:
- Vorschub des Metallblechs;
- Bereitstellung eines Fluidstromes durch die Fluidverteileinrichtung;
- Überwachungssignale von Komponenten der Vorrichtung;
- Gestalt des Metallblechs.

Die Abhängigkeit des kontrollierten Antriebs vom Vorschub des Metallblechs ist insbesondere dann zu gewährleisten, wenn das Konturrad als Transportorgan für den Vorschub des Metallblechs selbst verwendet wird. Das heißt mit anderen Worten, dass ein Antrieb des Kegelrades in der Weise erfolgt, dass der gewünschte Vorschub des Metallblechs eingehalten wird. Dies geschieht vorteilhafterweise intermittierend mit einer Taktfrequenz von insbesondere mehr als 20 Hz. Auch ist es möglich, den Antrieb des Kegelrades von zumindest einem Parameter des Fluidstroms abhängig gestalten, beispielsweise der anliegenden Strömungsgeschwindigkeit, des anliegenden Drucks, des anliegenden Volumenstromes bzw. der Form des Fluidstromes. Auch kann die Vorrichtung an verschiedenen Stellen Sensoren, Messfühler, etc. aufweisen, die bestimmte Messwerte erfassen und mit einem Referenzwert vergleichen. Daraus resultieren Überwachungssignale, die unter Umständen eine Variierung des Antriebs zur Folge haben können. Es ist auch möglich, dass das Metallblech in vorgegebenen Abständen bzw. Abschnitten unterschiedlich gestaltet sein soll, beispielsweise bei der Herstellung unterschiedlicher Varianten mit derselben Vorrichtung. Dabei kann es erforderlich sein, dass unterschiedliche Bearbeitungszeiten notwendig sind, so dass in Abhängigkeit von der Gestalt des Metallblechs unterschiedliche Vorschübe des Metallblechs bzw. Rotationsgeschwindigkeiten des Konturrades eingestellt werden müssen. Bevorzugt sind die Mittel in der Lage, den Antrieb des Konturrades in Abhängigkeit von mindestens zwei der obengenannten Faktoren, bevorzugt sogar aller Faktoren, zu kontrollieren bzw. zu regeln.

Außerdem wird auch vorgeschlagen, dass die Vorrichtung mit einer Vorrichtung zur Blechtrennung kombiniert ist. Dabei ist insbesondere auch gemeint, dass die Antriebe der Vorrichtung zur Blechtrennung und der Antriebe des Konturrades miteinander synchronisiert sind, so dass die Einbringung von Schlitzen, Öffnungen und dergleichen in das zu strukturierende Metallblech an die gewünschte Stelle des Konturrades bzw. der Gegenform gelangen, wenn ihnen die gewünschte Struktur verliehen wird.

Die Erfindung wird nachfolgend anhand der Figuren näher erläutert. Die Figuren zeigen besonders bevorzugte Ausgestaltungen sowie das Umfeld der Erfindung.

Es zeigen:
- Fig. 1:: ein Ausführungsbeispiel einer Fluidverteileinrichtung zur Herstellung ei- ner Struktur in ein Metallblech;
- Fig. 2:: eine Detailansicht der in Fig. 1 dargestellten Fluidverteileinrichtung;

- Fig. 3:: eine Ausführungsform einer Vorrichtung zur Durchführung eines Verfah- rens zur Herstellung einer Struktur in ein Metallblech;
- Fig. 4:: eine Detailansicht des Kontaktbereiches von Gegenform und Metallblech;
- Fig. 5:: schematisch und perspektivisch ein Ausführungsbeispiel für eine Abgas- behandlungskomponente; und
- Fig. 6:: verschiedene Ausführungsbeispiele von Strukturen in einem Metallblech.

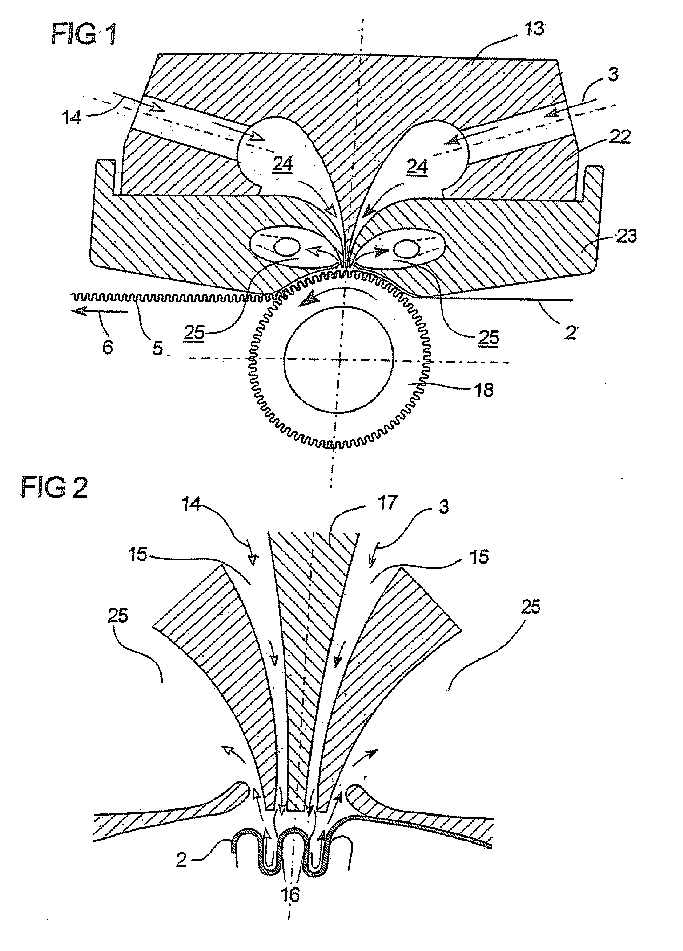

Fig. 1 zeigt schematisch und in einem Teilquerschnitt eine mögliche Ausführungsform einer Fluidverteileinrichtung 13 zur Herstellung eines Metallblechs 2 mit einer Struktur 5. Die Fluidverteileinrichtung 13 ist hier zweiteilig ausgebildet und weist ein Oberteil 22 und ein Unterteil 23 auf. Das Oberteil 22 ist mit zwei Einströmkanälen 24 versehen, wobei ein Einströmkanal 24 für einen ersten Fluidstrom 3 (gekennzeichnet durch die schwarzen Pfeile) und einem zweiten Einströmkanal 24 für einen zweiten Fluidstrom 14 (gekennzeichnet durch weiße Pfeile) versehen. Die beiden Einströmkanäle 24 laufen im Wesentlichen symmetrisch zu einem Mittelsteg 17. Bei der hier dargestellten Ausführungsvariante der Fluidverteileinrichtung 13 werden die Einströmkanäle 24 teilweise auch durch das Unterteil 23 begrenzt. Teilbereiche des Unterteils 23 bilden mit dem Mittelsteg 17 des Oberteils 23 Spaltdüsen 15 aus. An diesen Spaltdüsen 15 wird das Metallblech 2 vorbeigeführt und mit den Fluidströmen 3, 14 beaufschlagt. Dabei wird das Metallblech 2 in eine Gegenform 4, hier als Konturrad 18 dargestellt, gedrückt. Dabei sind auf gegenüberliegenden Seiten des Metallblechs 2 einerseits die Fluidverteileinrichtung 13 und andererseits die Gegenform, die hier als Konturrad 18 ausgebildet ist, angeordnet. Das Konturrad 18 dreht sich intermettierend, wobei eine Vorschubrichtung 6 des Metallblechs 2 realisiert wird.

Während der Bearbeitung strömen die Fluidströme 3, 14 hin zu dem Metallblech 2, drücken dieses in die Kontur des Konturrades 18, wobei die Fluidströme 3, 14 eine Umlenkung bezüglich ihrer Strömungsrichtung erfahren. Nach dem Kontakt mit dem Metallblech 2 strömen die Fluidströme in Auslasskanäle 25, die im Unterteil 23 der Fluidverteileinrichtung 13 vorgesehen sind. Ausgehend von diesen Auslasskanälen 25 werden die Fluidströme wieder aufbereitet und können somit der Fluidverteilerinrichtung 13 erneut zur Bearbeitung eines Metallblechs 2 zugeführt werden.

Fig. 2 zeigt schematisch eine Detailansicht der Düsen 15, die jeweils mit einem Spalt 16 ausgebildet sind. Wie dargestellt, strömen der erste Fluidstrom 3 (schwarze Pfeile) und der zweite Fluidstrom 14 (weiße Pfeile) zunächst hin zum Mittelsteg 17 und weiter in Richtung des jeweiligen Spaltes 16. Dort treten die Fluidströme 3, 14 aus und kommen in Kontakt mit dem darunter angeordneten Metallblech 2. Dabei drücken sie das Metallblech 2 in die Kontur des gegenüberliegend angeordneten Konturrades 18, wobei das Metallblech 2 zumindest teilweise eine plastische Umformung erfährt und die gewünschte Struktur 5 annimmt, die im wesentlichen der Kontur des Konturrades 18 entspricht. Aufgrund der Kontur bzw. der Struktur 5 wird eine Strömungsumlenkung der Fluidströme bewirkt, wobei diese in ihren jeweiligen Auslasskanal 25 geleitet werden. Die Anordnung der Düsen 15 und der Auslasskanäle 25 erfolgt in Übereinstimmung mit den durch die Struktur 5 bzw. Gegenform 4 bewirkten Strömungsumlenkungen. Auf diese Weise kann ein sehr großer Bestandteil des eingesetzten Fluidstromes 3,14 direkt nach dem Kontakt mit dem Metallblech 2 wieder entfernt werden.

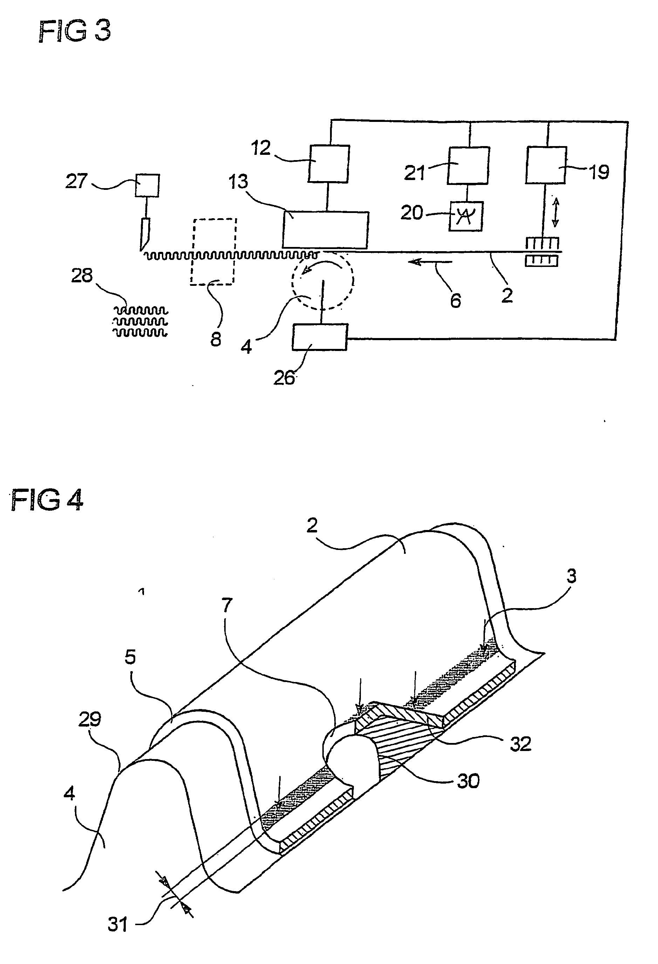

Fig. 3 zeigt schematisch einen möglichen Aufbau für eine Fertigungsanlage zur Herstellung von Abgasbehandlungskomponenten, wobei auch die erfindungsgemäße Vorrichtung zur Erzeugung einer Struktur 5 in einem Metallblech 2 umfasst ist. Das Metallblech 2 wird in Vorschubrichtung 6 zunächst einer Blechtrennungsvorrichtung 19 zugeführt. Der Vorschub des Metallbleches 2 wird hier durch die Gegenform 4 erzeugt. Die Blechtrennungsvorrichtung 19 erzeugt Schlitze 7, Öffnungen 40 oder sonstige Aussparungen im Material das Metallblechs 2. Nachfolgend wird das Metallblech 2 an einem Sensor 20 vorbeigeführt, der beispielsweise die Funktionalität der Blechtrennungsvorrichtung 19 überwacht. Die mit dem Sensor 20 gewonnenen Signale werden an eine Auswerteeinheit 21 weitergegeben.

Durch die Rotation der Gegenform 4 wird das Metallblech 2 nun weiterhin zur Fluidverteileinrichtung 13 hin bewegt. Dazu ist die Gegenform 4 mit einem Antrieb 26 ausgestattet, der einen intermittierenden Vorschub des Metallblechs 2 ermöglicht. Der Fluidverteileinrichtung 13 wird durch eine Fluidbereitstellungsvorrichtung 12 das zur Bearbeitung des Metallblechs 2 erforderliche Fluid zur Verfügung gestellt. Dieses drückt das zuvor glatte Metallblech 2 in die Kontur bzw. Gegenform 4, so dass das Metallblech 2 eine plastische Umformung erfährt.

Das nun strukturierte Metallblech 2 wird weiter zu einer Reinigungsvorrichtung 8 transportiert, wo beispielweise Reste des Fluides und Verunreinigungen entfernt werden. Die Reinigungsvorrichtung 8 kann als Durchlaufofen, Gebläse oder einer Kombination von beiden ausgeführt sein. Das gereinigte und getrocknete strukturierte Metallblech 2 wird nun abschließend einer Schneidevorrichtung 27 zugeführt, die aus dem als Band ausgeführten Metallblech einzelne Folien 28 abtrennt. Bei der dargestellten Anlage übernimmt die Auswerteeinheit 21 die Regelung verschiedener Komponenten, insbesondere der Fluidbereitstellungsvorrichtung 12, der Blechtrennvorrichtung 19 und des Antriebes 26 der Gegenform 4, die hier gleichzeitig als Transportorgan ausgeführt ist.

Fig. 4 zeigt schematisch und in einer Detailansicht ein Ausführungsbeispiel für eine Gegenform 4 sowie ein sich daran anschmiegendes Metallblech 2. Die Gegenform 4 weist einerseits eine Primärkontur 29 sowie eine diese überlagernde Sekundärstruktur 30 auf. Das Metallblech 2 wurde zunächst mit einer Blechtrennungsvorrichtung 19 geschlitzt, so dass in inneren Bereichen des Metallbleches 2 Schlitze 7 gebildet sind. Die Schlitze 7 sind in Übereinstimmung zu den Sekundärkonturen 30 der Gegenform 4 angeordnet. Der erste Fluidstrom 3, der hier maßgeblich die Umformung des Metallblechs 2 bewirkt, ist zeilenförmig ausgebildet und weist eine Zeilenbreite 31 auf, die bevorzugt kleiner 0,5 mm beträgt. Infolge des Auftreffens des ersten Fluidstromes 3 auf das Metallblech 2 wird einerseits eine der Primärkontur 29 entsprechende Struktur 5 ausgebildet, zusätzlich wird aber auch eine Ausstülpung 32 hergestellt, die durch die Vorsehung des Schlitzes 7 und die Sekundärkontur 30 geformt wird.

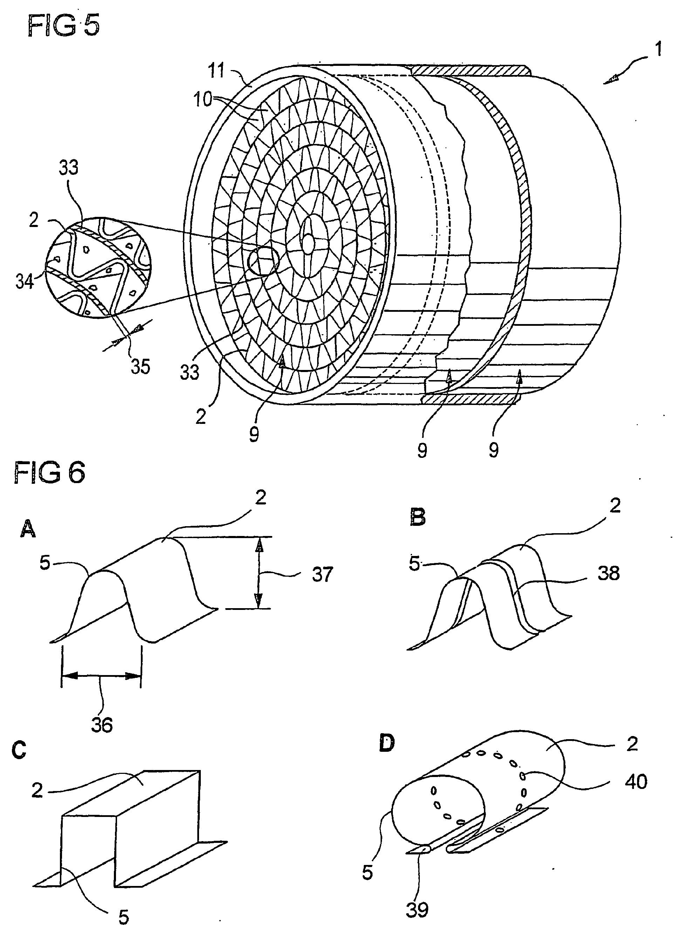

Fig. 5 zeigt ein Ausführungsbeispiel einer Abgasbehandlungskomponente 1, wie sie schließlich nach der Durchführung des Verfahrens vorliegen kann. Die Abgasbehandlungskomponente 1 weist ein Gehäuse 11 auf, das gleichzeitig drei hintereinander und beabstandet zueinander angeordnete Wabenkörper 9 umfasst. Die Wabenkörper 9 weisen eine Vielzahl für Abgas durchströmbare Kanäle 10 auf. Die Kanäle 10 werden durch ein strukturiertes Metallblech 2 sowie ein Glattband 33 gebildet. Aus der Vergrößerung lässt sich erkennen, dass das Glattband 33 als poröse Filterlage ausgeführt ist. Zusätzlich ist eine Beschichtung 34 sowohl auf dem Glattband 33 als auch auf dem Metallblech 2 vorgesehen. Das Metallblech 2 weist dabei eine Blechdicke 35 kleiner 110 µm auf.

Fig. 6 zeigt schematisch und in einer perspektivischen Darstellung vier unterschiedliche Beispiele für Strukturen 5 des Metallblechs 2. Die mit "A" gekennzeichnete Variante zeigt eine sinusähnliche Struktur 5, die sich durch eine Weite 36 und eine Höhe 37 beschreiben lässt. Bevorzugt sind dabei relativ schlanke Strukturen, bei denen das Verhältnis von Höhe zu Weite im Bereich kleiner 2 insbesondere im Bereiche von 1,5 bis 1,3.

Mit "B" ist eine Struktur 5 gekennzeichnet, die von einer zweiten, sogenannten Mikrostruktur 38 überlagert wird. Hier sind die Struktur 5 und die Mikrostruktur 38 im wesentlichen senkrecht zueinander ausgebildet, sie können aber auch schräg zueinander verlaufen.

Die Struktur 5 der Variante "C" weist einen im wesentlichen rechteckigen Verlauf auf. Die Variante nach "D" zeigt eine, zusätzlich Öffnungen 40 aufweisende, sogenannte Omega-Struktur mit Hinterschneidungen 39, wie sie üblicherweise durch Wälzverfahren nicht ohne weiteres herstellbar ist.

Das hier beschriebene Verfahren sowie die erfindungsgemäße Vorrichtung erlauben in besonders einfacher und kostengünstiger Weise die Herstellung verschiedenster Ausgestaltungen von Strukturen in Metallblechen. Dabei können diese Fertigungsschritte selbst den Anforderungen zur Herstellung von Abgasbehandlungskomponenten genügen, was bislang nicht in Betracht gezogen wurde.

### Bezugszeichenliste

- 1: Abgasbehandlungskomponente
- 2: Metallblech
- 3: Erster Fluidstrom
- 4: Gegenform
- 5: Struktur
- 6: Vorschubrichtung
- 7: Schlitz
- 8: Reinigungsvorrichtung
- 9: Wabenkörper
- 10: Kanal
- 11: Gehäuse
- 12: Fluidbereitstellungsvorrichtung
- 13: Fluidverteileinrichtung
- 14: Zweiter Fluidstrom
- 15: Düse
- 16: Spalt
- 17: Mittelsteg
- 18: Konturrad
- 19: Blechtrennungsvorrichtung
- 20: Sensor
- 21: Auswerteeinheit
- 22: Oberteil
- 23: Unterteil
- 24: Einströmkanal
- 25: Ausströmkanal
- 26: Antrieb
- 27: Schneidevorrichtung
- 28: Folie
- 29: Primärkontur
- 30: Sekundärkontur
- 31: Zeilenbreite
- 32: Ausstülpung
- 33: Glattband
- 34: Beschichtung
- 35: Blechdicke
- 36: Weite
- 37: Höhe
- 38: Mikrostruktur
- 39: Hinterschneidung
- 40: Öffnung

## Patentansprüche

1. Verfahren zur Herstellung einer Abgasbehandlungskomponente (1) umfassend mindestens ein strukturiertes Metallblech (2), bei dem mindestens ein Metallblech (2) mittels wenigstens einem Fluidstrom (3,14) umgeformt wird, um das mindestens eine strukturierte Metallblech (2) zu bilden, **dadurch gekennzeichnet, dass** das mindestens eine strukturierte Metallblech (2) so angeordnet wird, dass ein Wabenkörper (9) gebildet wird, der eine Vielzahl von Kanälen (10) aufweist.

2. Verfahren nach Anspruch 1, bei dem der mindestens eine Fluidstrom (3,14) zumindest einen der folgenden Bestandteile aufweist:
- Wasser,
- Öl,
- Partikel,
- Additive.

3. Verfahren nach Anspruch 1 oder 2, bei dem das Verfahren intermittierend abläuft.

4. Verfahren nach einem der vorstehenden Ansprüche, bei dem das mindestens eine Metallblech (2) vor der Umformung einem Verfahrensschritt umfassend eine Blechtrennung unterzogen wird.

5. Verfahren nach einem der vorstehenden Ansprüche, bei dem ein im wesentlichen glattes Metallblech (2) durch den wenigstens einen Fluidstrom (3,14) in mindestens eine Gegenform (4) gedrückt wird, so dass sich die Struktur (5) des mindestens einen strukturierten Metallblechs (2) ausbildet.

6. Verfahren nach Anspruch 5, bei dem mindestens zwei Fluidströme eingesetzt werden, wobei ein erster Fluidstrom (3) eine Blechumformung und ein nachgeschalteter zweiter Fluidstrom (14) eine Fixierung des Metallblechs (2) bezüglich der mindestens einen Gegenform (4) bewirkt.

7. Verfahren nach Anspruch 5 oder 6, bei dem die mindestens eine Gegenform (4) gleichzeitig das mindestens eine strukturierte Metallblech (2) in eine Vorschubrichtung (6) transportiert.

8. Verfahren nach einem der Ansprüche 5 bis 7, bei dem in einem der Blechumformung vorgelagerten Verfahrensschritt mittels Blechtrennung eine Mehrzahl von Schlitzen (7) in dem mindestens einen Metallblech (2) erzeugt werden, wobei eine kontrollierte Positionierung der Schlitze (7) gegenüber der Gegenform (4) durchgeführt wird.

9. Verfahren nach einem der vorstehenden Ansprüche, bei dem das mindestens eine strukturierte Metallblech (2) nach dem Umformen einem Reinigungsprozess unterzogen wird.

10. Verfahren nach einem der vorstehenden Ansprüche, bei dem der Wabenkörper (10) mit einem Gehäuse (11) in Kontakt gebracht und anschließend ein thermischer Fügeprozess durchgeführt wird.

11. Vorrichtung zur Herstellung einer Struktur (5) in ein Metallblech (2) zur Herstellung einer Abgasbehandlungskomponente gemäß dem Verfahren nach einem der Ansprüche 1 bis 10, umfassend eine Fluidbereitstellungsvorrichtung (12), mindestens eine Fluidverteileinrichtung (13) und mindestens eine Gegenform (4), wobei die mindestens eine Fluidverteileinrichtung (13) zumindest einen ersten Fluidstrom (3) und einen zweiten Fluidstrom (14) bereitstellt, **dadurch gekennzeichnet, dass** die Vorrichtung mit mindestens einem Sensor (20) und wenigstens einer Auswerteeinheit (21) ausgestattet ist, wobei die Auswerteeinheit (21) die Regelung der Fluidbereitstellungsvorrichtung (12) und des Antriebes(26) der Gegenform (4) übernimmt.

12. Vorrichtung nach Anspruch 11, **dadurch gekennzeichnet, dass** die mindestens eine Fluidverteileinrichtung (13) Mittel zur Variierung wenigstens einem der folgenden Parameter hinsichtlich zumindest des ersten Fluidstroms (3) oder des zweiten Fluidstroms (14) hat:
a) Druck,
b) Strömungsgeschwindigkeit,
c) Volumenstrom,
d) Form.

13. Vorrichtung nach Anspruch 11 oder 12, **dadurch gekennzeichnet, dass** die mindestens eine Fluidverteileinrichtung (13) wenigstens eine Düse (15) mit einem Spalt (16) umfast.

14. Vorrichtung nach einem der Ansprüche 11 bis 13, **dadurch gekennzeichnet, dass** die mindestens eine Fluidverteileinrichtung (13) zwei, von einem Mittelsteg (13) gegenüberliegende Düsen (15) mit jeweils einem Spalt (16) aufweist.

15. Vorrichtung nach einem der Ansprüche 11 bis 14, **dadurch gekennzeichnet, dass** die mindestens eine Fluidverteileinrichtung (13) Mittel zum Entfernen des Fluides nach dem Kontakt mit dem Metallblech (2) umfasst.

16. Vorrichtung nach einem der Ansprüche 11 bis 15, **dadurch gekennzeichnet, dass** die mindestens eine Gegenform (4) eine drehbares Konturrad (18) ist.

17. Vorrichtung nach Anspruch 16, **dadurch gekennzeichnet, dass** diese Vorrichtung Mittel zum kontrollierten Antrieb des Konturrades (18) in Abhängigkeit wenigstens einem der folgenden Faktoren hat:
- Vorschub des Metallblechs (2);
- Bereitstellung eines Fluidstroms (3,14) durch die Fluidverteileinrichtung (13);
- Überwachungssignale von Komponenten der Vorrichtung;
- Gestalt des Metallblechs (2).

18. Vorrichtung nach einem der Ansprüche 11 bis 15, **dadurch gekennzeichnet, dass** diese mit einer Blechtrennungsvorrichtung (19) kombiniert ist.

## Claims

1. A method for manufacturing an exhaust gas treatment component (1) comprising at least one structured piece of sheet metal (2), in which the at least one piece of sheet metal (2) is shaped by means of at least one fluid stream (3, 14) to form the at least one structured piece of sheet metal (2), **characterized in that** the at least one structured piece of sheet metal (2) is arranged in such a way that a honeycomb element (9) is formed which has a plurality of cells (10).

2. The method as claimed in claim 1, in which the at least one fluid stream (3, 14) has at least one of the following components:
- water,
- oil,
- particles,
- additive.

3. The method as claimed in claim 1 or 2, in which the method takes place intermittently.

4. The method as claimed in one of the preceding claims, in which, before the shaping, the at least one piece of sheet metal (2) is subjected to a method step comprising the cutting of sheet metal.

5. The method as claimed in one of the preceding claims, in which an essentially smooth piece of sheet metal (2) is pressed by the at least one fluid stream (3, 14) into at least one countermold (4) so that the structure (5) of the at least one structured piece of sheet metal (2) is formed.

6. The method as claimed in claim 5, in which at least two fluid streams are used, a first fluid stream (3) bringing about shaping of sheet metal and a subsequent, second fluid stream (14) causing the piece of sheet metal (2) to be fixed with respect to the at least one countermold (4).

7. The method as claimed in claim 5 or 6, in which the at least one countermold (4) simultaneously transports the at least one structured piece of sheet metal (2) in a forward feed direction (6).

8. The method as claimed in one of claims 5 to 7, in which, in a method step which precedes the shaping of sheet metal, a plurality of slits (7) are made in the at least one piece of sheet metal (2) by cutting sheet metal, wherein the slits (7) with respect to the countermold (4) are positioned in a controlled fashion.

9. The method as claimed in one of the preceding claims, in which the at least one structured piece of sheet metal (2) is subjected to a cleaning process after shaping.

10. The method as claimed in any of the preceding claims, in which the honeycomb element (10) is placed in contact with a housing (11) and a thermal joining process is subsequently carried out.

11. A device for producing a structure (5) in a piece of sheet metal (2) for manufacturing an exhaust gas treatment component (1) according to the method as claimed in any of the claims 1 to 10, comprising a fluid supply device (12), at least one fluid distribution device (13) and at least one countermold (4), wherein the at least one fluid distribution device (13) conditions at least a first fluid stream (3) and a second fluid stream (14), **characterized in that** said device is equipped with at least one sensor (20) and at least one evaluation unit (21), wherein the evaluation unit (21) assumes the function of controlling the fluid supply device (12) and a drive (26) of the countermold (4).

12. The device as claimed in claim 11, **characterized in that** the at least one fluid distribution device (13) has means for varying at least one of the following parameters with respect to at least the first fluid stream (3) or the second fluid stream (14):
a) pressure,
b) flow rate,
c) volume flow,
d) shape.

13. The device as claimed in claim 11 or 12, **characterized in that** the at least one fluid distribution device (13) comprises at least one nozzle (15) with a gap (16).

14. The device as claimed in one of claims 11 to 13, **characterized in that** the at least one fluid distribution device (13) has two nozzles (15) which lie opposite a center web (13) and each have a gap (16).

15. The device as claimed in one of claims 11 to 14, **characterized in that** the at least one fluid distribution device (13) comprises means for removing the fluid after contact with the piece of sheet metal (2).

16. The device as claimed in one of claims 11 to 15, **characterized in that** the at least one countermold (4) is a rotatable contoured wheel (18).

17. The device as claimed in claim 16, **characterized in that** this device has means for driving the contoured wheel (18) in a controlled fashion as a function of at least one of the following factors:
- forward feed of the piece of sheet metal (2);
- supplying of a fluid stream (3, 14) by the fluid distribution device (13);
- monitoring signals of components of the device;
- shape of the piece of sheet metal (2).

18. The device as claimed in one of claims 11 to 15, **characterized in that** said device is combined with a sheet metal cutting device (19).

## Revendications

1. Procédé destiné à la fabrication d'un composant de traitement de gaz d'échappement (1) comprenant au moins une tôle métallique structurée (2), dans le cas duquel au moins une tôle métallique (2) est déformée au moyen d'au moins un flux de fluide (3,14), pour former l'au moins une tôle métallique structurée (2), **caractérisé en ce que** l'au moins une tôle métallique structurée (2) est agencée de façon telle qu'un corps en nids d'abeilles (9) est formé, qui a une multiplicité de canaux (10).

2. Procédé selon la revendication 1, dans lequel l'au moins un flux de fluide (3, 14) a au moins un des constituants suivants :
- de l'eau,
- de l'huile
- des particules
- des additives.

3. Procédé selon les revendications 1 ou 2, dans lequel le procédé se déroule de manière intermittente.

4. Procédé selon l'une des revendications précédentes, dans lequel avant d'être déformée l'au moins une tôle métallique (2) est soumise à une étape de procédé, comprenant une séparation de tôle.

5. Procédé selon l'une des revendications précédentes, dans lequel une tôle métallique (2) sensiblement lisse est pressée par l'au moins un flux de fluide (3, 14) dans au moins un contre-moule (4), de sorte que la structure (5) de l'au moins une tôle métallique structurée (2) se forme.

6. Procédé selon la revendication 5, dans lequel au moins deux flux de fluide sont utilisés, un premier flux de fluide (3) provoquant une déformation de la tôle et un deuxième flux de fluide (14) branché en aval, provoquant une fixation de la tôle métallique (2) quant à l'au moins un contre-moule (4).

7. Procédé selon la revendication 5 ou 6, dans lequel l'au moins un contre-moule (4) transporte l'au moins une tôle métallique structurée (2) dans une direction d'avancement (6).

8. Procédé selon l'une des revendications 5 à 7, dans lequel dans une étape du procédé, située en amont de la déformation de la tôle, une pluralité de fentes (7) étant générée dans l'au moins une tôle métallique (2) au moyen de la séparation de tôles, un positionnement contrôlé des fentes (7) par rapport au contre-moule (4) étant effectué.

9. Procédé selon l'une des revendications précédentes, dans lequel après la déformation l'au moins une tôle métallique structurée (2) est soumise à un processus d'épuration.

10. Procédé selon l'une des revendications précédentes, dans lequel le corps en nids d'abeilles (10) est mis en contact avec un boîtier (11) et un processus thermique de jointoiement est ensuite effectué.

11. Dispositif pour la fabrication d'une structure (5) en une tôle métallique (2) pour fabriquer un composant de traitement de gaz d'échappement conforme au procédé selon l'une des revendications 1 à 10, comprenant un dispositif de mise à disposition de fluide (12), au moins un dispositif de répartition de fluide (13) et au moins un contre-moule (4), l'au moins un dispositif de répartition de fluide (13) met à disposition au moins un premier flux de fluide (3) et un deuxième flux de fluide (14), **caractérisé en ce que** le dispositif est équipé avec au moins un capteur (20) et au moins avec une unité d'évaluation (21), l'unité d'évaluation (21) prenant en charge le réglage du dispositif de mise à disposition de fluide (12) et de l'entraînement (26) du contre-moule (4).

12. Dispositif selon la revendication 11, **caractérisé en ce que** l'au moins un dispositif de répartition de fluide (13) a des moyens pour varier au moins un des paramètres suivants au moins quant au premier flux de fluide (3) ou au deuxième flux de fluide (14) :
a) pression,
b) vitesse d'écoulement,
c) flux du volume
d) forme

13. Dispositif selon la revendication 11 ou 12, **caractérisé en ce que** l'au moins un dispositif de répartition de fluide (13) comprend au moins une buse (15) avec une fente (16).

14. Dispositif selon l'une des revendications 11 à 13, **caractérisé en ce que** l'au moins un dispositif de répartition de fluide (13) dispose de deux buses qui sont opposées (15) à partir d'une entretoise (13), les buses ayant respectivement une fente (16).

15. Dispositif selon l'une des revendications 11 à 14, **caractérisé en ce que** l'au moins un dispositif de répartition de fluide (13) comprend des moyens pour supprimer le fluide après le contact avec la tôle métallique (2).

16. Dispositif selon l'une des revendications 11 à 15, **caractérisé en ce que** l'au moins un contre-moule (4) est une roue profilée rotative (18).

17. Dispositif selon la revendication 16, **caractérisé en ce que** ce dispositif a des moyens pour l'entraînement contrôlé de la roue profilée (18) en fonction d'au moins un des facteurs suivants :
- avancement de la tôle métallique (2);
- mise à disposition d'un flux de fluide (3, 14) par le dispositif de répartition de fluide (13);
- des signaux de contrôle de composants du dispositif;
- configuration de la tôle métallique (2).

18. Dispositif selon l'une des revendications 11 à 15, **caractérisé en ce que** celui-ci est combiné avec un dispositif de séparation de tôles (19).
